(19)

Europäisches
Patentamt
European
Patent Office
Office européen
des brevets

(11)  **EP 1 448 165 B1**

(12)  **EUROPEAN PATENT SPECIFICATION**

(45) Date of publication and mention
of the grant of the patent:
**19.09.2007 Bulletin 2007/38**

(21) Application number: **02774211.3**

(22) Date of filing: **13.11.2002**

(51) Int Cl.:
***A61K 9/00*** (2006.01)

(86) International application number:
**PCT/CA2002/001727**

(87) International publication number:
**WO 2003/041682 (22.05.2003 Gazette 2003/21)**

(54) **LIPID CARRIER COMPOSITIONS AND METHODS FOR IMPROVED DRUG RETENTION**

LIPIDTRÄGERZUSAMMENSETZUNGEN UND VERFAHREN ZUR VERBESSERTEN
WIRKSTOFFZURÜCKHALTUNG

COMPOSITIONS A VECTEURS LIPIDIQUES ET PROCEDES GARANTISSANT UNE MEILLEURE
RETENTION MEDICAMENTEUSE

(84) Designated Contracting States:
**AT BE BG CH CY CZ DE DK EE ES FI FR GB GR
IE IT LI LU MC NL PT SE SK TR**

(30) Priority: **13.11.2001 US 331249 P**

(43) Date of publication of application:
**25.08.2004 Bulletin 2004/35**

(73) Proprietor: **Celator Pharmaceuticals, Inc.
Princeton, NJ 08540 (US)**

(72) Inventors:
• **WEBB, Murray
British Columbia V7R 3C6 (CA)**
• **TARDI, Paul
Surrey, British Columbia V3S 7M6 (CA)**

(74) Representative: **Vossius & Partner
Siebertstrasse 4
81675 München (DE)**

(56) References cited:
**WO-A-01/05372        WO-A-90/15595
WO-A-91/04019        WO-A-95/15762
US-A- 4 927 571       US-A- 5 393 530
US-A- 5 882 679       US-A- 5 993 850
US-A- 6 106 858       US-B1- 6 270 806
US-B1- 6 306 432**

• **BONDURANT B ET AL: "Photoinitiated
destabilization of sterically stabilized liposomes"
BIOCHIMICA ET BIOPHYSICA ACTA.
BIOMEMBRANES, AMSTERDAM, NL, vol. 1511,
no. 1, 9 March 2001 (2001-03-09), pages 113-122,
XP004273405 ISSN: 0005-2736**

• **GABER M H ET AL: "THERMOSENSITIVE
STERICALLY STABILIZED LIPOSOMES:
FORMULATION AND IN VITRO STUDIES ON
MECHANISM OF DOXORUBICIN RELEASE BY
BOVINE SERUM AND HUMAN PLASMA"
PHARMACEUTICAL RESEARCH, NEW YORK,
NY, US, vol. 12, no. 10, 1995, pages 1407-1416,
XP000979539 ISSN: 0724-8741 cited in the
application**

• **KENWORTHY A K ET AL: "Structure and phase
behavior of lipid suspensions containing
phospholipids with covalently attached poly
(ethylene glycol)." BIOPHYSICAL JOURNAL.
UNITED STATES MAY 1995, vol. 68, no. 5, May
1995 (1995-05), pages 1903-1920, XP009012613
ISSN: 0006-3495 cited in the application**

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

**Description**

<u>Cross-Reference to Related Applications</u>

This application claims benefit of U.S. Serial No. 60/331,249, and U.S. Serial No. 60/331,248 both filed 13 November 2001.

<u>Technical Field</u>

This invention is directed to improving drug retention in lipid-based therapeutic carrier systems by maintaining low osmotic pressure of the internal aqueous medium.

<u>Background Art</u>

Over the last decade significant progress has been made in the clinical development of liposomes for drug delivery of anti-cancer agents. Although chemotherapeutic agents are effective, there is significant toxicity to normal cells resulting in symptoms including nausea, alopecia, myelosuppression, cardio- and nephrotoxicity. Encapsulation of anti-cancer agents in drug delivery systems such as liposomes has proven to be beneficial because drug exposure to normal cells can be drastically reduced resulting in significantly lower toxic side effects.

Liposomes are made up of one or more lipid bilayers enclosing an internal compartment. Liposomes can be categorized into multilamellar vesicles, multivesicular liposomes, unilamellar vesicles and giant liposomes. Multilamellar liposomes (also known as multilamellar vesicles or "MLV") contain multiple concentric bilayers within each liposome particle, resembling the "layers of an onion." Multivesicular liposomes consist of lipid membranes enclosing multiple non-concentric aqueous chambers. Unilamellar liposomes (also known as unilamellar vesicles or "ULV") enclose a single internal aqueous compartment and are classified as either small unilamellar vesicles (SUV) or large unilamellar vesicles (LUV). LUV and SUV range in size from about 500 to 50 nm and 50 to 20 nm, respectively. The *in vivo* use of SUV has been limited, because of a number of drawbacks. Giant liposomes typically range in size from 5000 nm to 50,000 nm and are used mainly for studying mechanochemical and interactive features of lipid bilayer vesicles *in vitro.*

In order for therapeutic effectiveness of liposome encapsulated drugs to be realized, such drugs must be effectively retained within a liposome after intravenous administration and the liposomes must have a sufficient circulation lifetime to permit the desired drug delivery.

Classical means of entrapping drugs (known as loading) into liposomes involves encapsulating the desired drug during the preparation of the liposomes (passive entrapment). Efficiency is often low because encapsulation strongly depends on the trapped volume of the liposomes.

An advancement in liposome loading techniques was the discovery that an ion gradient can be generated across a liposome membrane in order to actively load an ionizable drug (U.S. patent Nos. 5,736,155; 5,077,056; and 5,762,957). This method involves establishing a pH gradient across a liposome bilayer such that an ionizable drug to be encapsulated within a liposome is uncharged in the external buffer and charged within the aqueous interior. This allows the drug to readily cross the liposomal bilayer in the neutral form and to be trapped within the aqueous interior of the liposome due to conversion to the charged form. The most common method of loading agents with ionizable amine groups employs an internal buffer composition such as citrate, pH 4.0 and a neutral exterior buffer; however, other methods of establishing a pH gradient have also been used. Generally, the internal buffer concentrations employed for loading of drug are between 300 and 600 mM; although concentrations as low as 100 mM have been reported (U.S. patent No. 5,762,957).

Leakage of drug from actively loaded liposomes has been found to follow the loss of the proton gradient. U.S. patent No. 5,736,155 reported that elimination of the pH gradient across the liposomal membrane dramatically increased the rate of efflux of doxorubicin from liposomes. Thus, one way to assure retention of an active agent within the liposomes has been to maintain sufficient buffer strength in the internal solution to maintain the pH gradient.

An alternative approach to enhancing retention time of active biological agents within liposomes under physiological conditions has been the inclusion of a stabilizing agent such as cholesterol in the structure of the liposome. It has long been established that incorporation of membrane rigidification agents, such as cholesterol, into a liposomal membrane enhances circulation lifetime of the liposome as well as retention of drugs within the liposome. Inclusion of cholesterol in liposomal membranes has been shown to reduce release of drug after intravenous administration. Generally, cholesterol increases bilayer thickness and fluidity while decreasing membrane permeability, protein interactions, and lipoprotein destabilization of the liposome. For example, it has been reported that including increasing amounts of cholesterol in phosphatidylcholine liposomes decreased the leakage of calcein (a fluorescent marker compound) from liposomes in the presence and absence of an osmotic gradient (Allen, et al., Biochim. Biophys. Acta (1980) 418-426).

Conventional approaches to liposome formulation dictate inclusion of substantial amounts (*e.g.*, 30-45 mol %) of cholesterol or equivalent membrane rigidification agents (such as other sterols). An exception is described in PCT application, PCT/CA01/00655, which discloses that certain drugs that previously exhibited poor retention in cholesterol-containing liposomes, exhibited better drug retention in liposomes containing substantially no cholesterol. However, the conditions through which improved drug retention by these liposomes were not identified.

When considering the effects of cholesterol on liposome permeability Gaber, et al. (Pharm Res (1995) 10: 1407-1416) have shown that addition of cholesterol to gel phase lipids can increase entrapped content release in the presence of proteins. These investigators believed that this result was consistent with earlier biophysical studies showing that cholesterol affects the order parameter of the phospholipid acyl chains within the bilayer and this, in turn, effects membrane permeability. Under isosmotic conditions, cholesterol shows a stabilizing effect when the phospholipids used are in the liquid crystalline state, with consequently lower content leakage. In the gel state (a temperature below the transition temperature of the lipids used) cholesterol addition enhances content release. These investigators recognized, based on the biophysical properties of phospholipid membranes that cholesterol addition will modulate permeability properties.

It is well understood that removal of cholesterol from membranes prepared with lipids exhibiting a defined phase transition temperature (Tc) will result in improved content retention when the incubation temperature is below the Tc. However, the ideal behavior of liposomes prepared with substantially no cholesterol is compromised in the presence of serum proteins. Gaber, *et al.,* noted that liposomes prepared with substantially no cholesterol could be stabilized against the effects of serum by incorporating PEG-modified lipids, specifying however that cholesterol was still needed to stabilize these liposomes and provide optimal retention characteristics for formulations designed for intravenous use. Gaber, *et al.,* refer to earlier studies describing the destabilizing effects of specific serum proteins such as those responsible for the transfer of phosphatidylcholine to HDL, citing work from their own laboratory indicating that cholesterol was required to enhance the antitumor activity of liposomal formulations of cytosine arabinoside. Gaber, *et al.,* provide evidence suggesting that the destabilization of PEG-PE containing liposomes prepared with substantially no cholesterol was not due to complement, but due to other components in human plasma which had not been identified.

Thus Gaber, *et al.,* teach that optimal retention in liposomes designed for intravenous applications requires addition of cholesterol, even when using stabilizing lipids such as PEG-PE.

It is also understood that an osmotic gradient (hyperosmotic internal medium) can increase content release. Allen, et al. (Biochim. Biophys. Acta (1980) 418-426, cited above) demonstrate that incorporation of cholesterol reduced serum-induced leakage, and that leakage, from the cholesterol containing liposomes was greater when an osmotic gradient was present across the membrane.

Mui, et al., Biophys J. (1993) 64:443-453 demonstrated, using cholesterol-containing membranes, that osmotic gradient-induced lysis caused a gradual release of contents. When 100 nm vesicles were placed in a solution that was hypoosmotic with respect to the trapped intravesicular medium, the resulting influx of water caused the vesicles to assume a spherical shape, and osmotic differentials of sufficient magnitude produced membrane rupture that resulted in partial release of the intravesicular solutes. In further work, again using cholesterol-containing liposomes, Mui, et al. (J. Biol. Chem. (1994) 269:7364-7370) demonstrated that in both the presence and absence of plasma, lysis resulted in only partial loss of intravesicular solute; following membrane resealing the vesicle interior remained hyperosmotic with respect to the external medium.

When considering the influence of cholesterol on osmotic gradient-induced lysis, Mui, *et al.,* refer to early studies completed by Weinstein, *et al.,* indicating that serum protein interaction with dipalmitoylphosphatidylcholine induced complete release of entrapped contents in an all-or-nothing manner, and conclude that osmotic sensitivity will be dependent upon vesicle lipid composition. Mui, *et al.,* suggest that, in the absence of cholesterol, osmotic lysis would result in complete, as opposed to gradual, release of contents. It is recognized that in the absence of cholesterol, the presence of bilayer defects, such as the small-scale lipid structures identified by Jorgensen, et al. (Cell. Mol. Biol. Lett. (2001) 6:255-263), greatly favor protein insertion and solute release.

Thus, findings to date demonstrate that cholesterol is helpful to stabilize liposomes from plasma protein induced lysis and that, in the absence of cholesterol, the presence of membrane defects facilitates protein insertion. From these studies, it would be expected that in the presence of an osmotic gradient protein insertion, which would occur following intravenous administration, would result in complete, as opposed to gradual, loss of encapsulated contents. The present invention describes liposome compositions that, surprisingly, exhibit improved drug retention following intravenous administration, while containing low levels of (<20 mol%) or substantially no cholesterol and are prepared in solutions that exhibiting an osmolarity of less then 500 mOsm/kg (or an osmotic differential from physiological saline equal to or less than 200 mOsmlkg).

It has also been suggested that polyethyleneglycol (PEG) derivatized phosphatidyl ethanolamine can be used in place of cholesterol as a membrane-stabilizing component. For example, Blume, et al., Biochim. Biophys. Acta (1990) 1029:91-97 investigated the stability of liposomes containing distyryl phosphatidylcholine (DSPC) distearoyl phosphoethanolamine-PEG (DSPE-PEG) containing 100 mMol HEPES buffer pH 2 as an internal solution, but containing no active encapsulated compound *in vivo,* and suggested the substitution of PEG-coupled diacyl phosphatides as alternatives to cholesterol for stabilization. In a subsequent paper, Blume, et al., Biochim. Biophys. Acta (1993) 1146:157-168 again used liposomes containing no active biological ingredient *in vivo* to study the effects of various concentrations of DSPE-PEG. In both papers, *in vitro* experiments involved encapsulation of carboxyfluorescein, rather than a biologically active agent. Other studies involving the effect of PEGylated DSPE or PEG *per se* on liposomal structure where the liposomes do not contain biologically active agents but low concentration buffers as internal solutions are those of Kenworthy, et al., Biophys. J. (1995) 68:1903-1920; Belsito, et al., Biophys. J. (2001) 93:11-22; and Yamazaki, et al., Biophys. Chem. (1992) 43:29-37. Other papers describing the effect of inclusion of PEG include those of Maruyama, et al., Biochim. Biophys. Acta (1992) 1128(1): 44-49; Maruyama, et al., Chem. Pharm. Bull (Tokyo) (1991) 39(6):1620-1622; and Bedu-Addo, et al., Pharm. Res. (1996) 13(5):710-717.

Thus, the art does not describe liposomes substantially free of cholesterol, but containing alternative aggregation preventing agents, and containing a biologically active agent in an internal solution of osmolality less than 500 mOsm/kg, and there is no suggestion in the art that such liposomes would exhibit enhanced retention of the biological agent under physiological conditions.

Disclosure of the Invention

This invention is based on the finding that liposomes substantially free of cholesterol provide increased systemic retention of biologically active agents contained therein when the internal medium of the liposomes has an osmolarity of less than 500 mOsm/kg or an osmotic differential from physiological saline equal to or less than 200 mOsm/kg. Liposomes substantially free of cholesterol exhibit unanticipated improvements in the retention of encapsulated contents following intravenous administration.

Preferably, the liposomes are large unilamellar vesicles (LUV). In one embodiment, they comprise a hydrophilic polymer(s) grafted onto the surface by conjugation to a vesicle-forming lipid. They contain components that prevent aggregation and surface-surface interactions, such as phosphatidylglycerol, phosphatidylinositol and/or PEG modified lipids. In one embodiment the liposomes have a transition temperature > 38°C.

As discussed herein, the invention provides liposomes having drug retention properties suitable for administration to mammals, and thus includes pharmaceutical formulations comprising the liposomes of the invention, along with at least one pharmaceutically acceptable carrier.

The invention also relates to methods of administering liposomes to a mammal, and methods of treating a mammal affected by, susceptible to, or suspected of being affected by a disorder (*e.g.*, cancer). Methods of treatment and/or administration may optionally further comprise a step of selecting or identifying a mammal, preferably a human, affected by, susceptible to, or suspected of being affected by a disorder. Methods of treatment or of administration will generally be understood to comprise administering the pharmaceutical composition at a dosage sufficient to ameliorate said disorder or symptoms thereof

Brief Description of the Drawings

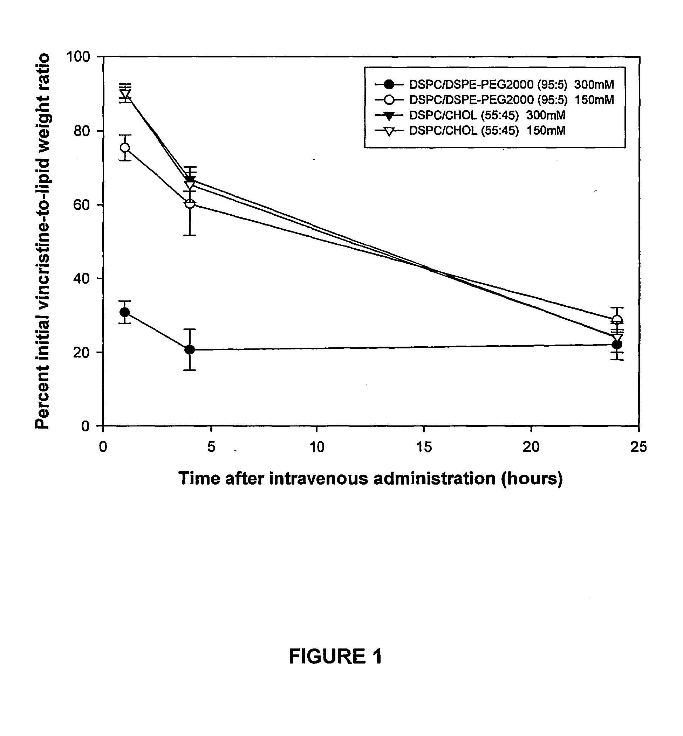

**Figure 1**: A graph showing the percent initial vincristine-to-lipid weight ratio (initial drug-to-lipid weight ratio was 0.1:1) in the blood after intravenous injection of Balb/c mice at various time points for liposomes consisting of DSPC/DSPE-PEG2000 (95:5 mole ratio) utilizing 300 mM citrate (filled circles) and 150 mM (open circles) as the internal loading buffer (about 600 and 300 mOsm/kg, respectively) and liposomes consisting of DSPC/cholesterol (55:45 mole ratio) utilizing 300 mM citrate (filled triangles) and 150 mM citrate (open triangles) as the internal loading buffer.

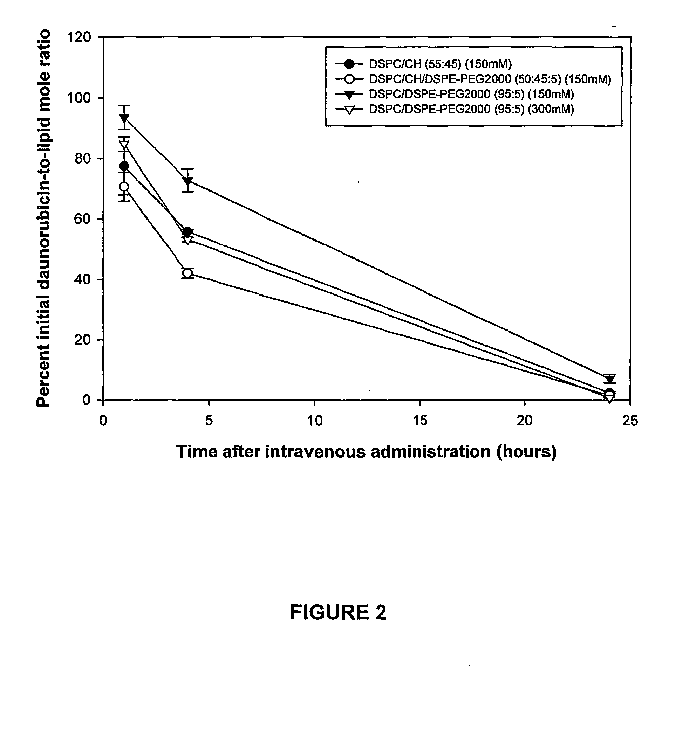

**Figure 2:** A graph showing the percent initial ratio of daunorubicin-to-lipid (initial drug-to-lipid mole ratio was 0.2:1) remaining in the blood after intravenous injection of Balb/c mice as a function of time for liposomes consisting of DSPC/cholesterol (55:45 mole ratio, filled circles), DSPC/cholesterol/DSPE-PEG2000 (50:45:5 mole ratio, open circles) and DSPC/DSPE-PEG2000 (95:5 mole ratio), utilizing either 150 (filled triangles) or 300 mM (open triangles) citrate, pH 4 (300 or 600 mOsm/kg, respectively) as the internal buffer.

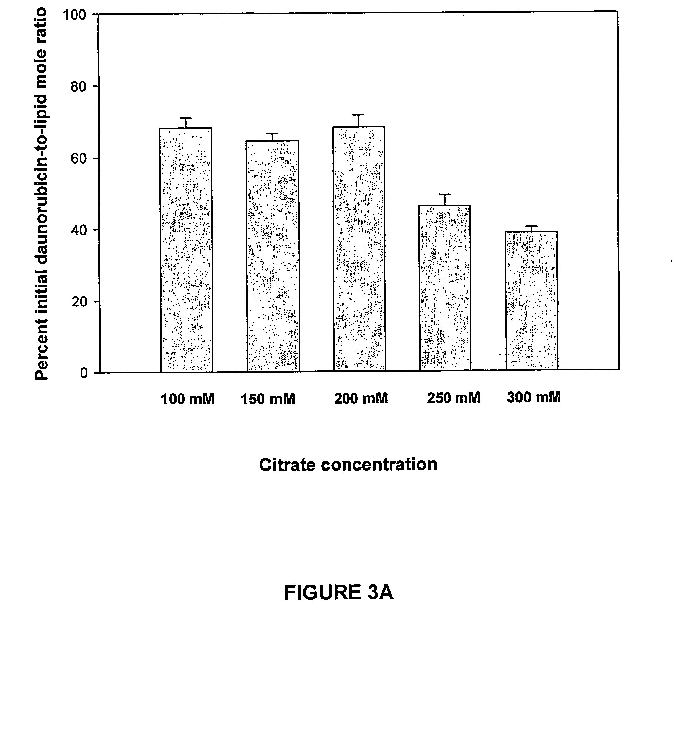

**Figure 3A:** A histogram showing the percent initial daunorubicin-to-lipid ratio (initial drug-to-lipid mole ratio was 0.2:1) remaining in the blood 4 hours after intravenous injection of Balb/c mice with liposomes consisting of DSPC/DSPE-PEG2000 (95:5 mole ratio) utilizing 100 mM, 150 mM, 200 mM, 250 mM and 300 mM citrate (200, 300, 400, 500 and 600 mOsm/kg, respectively), pH 4.0 as the internal loading buffer.

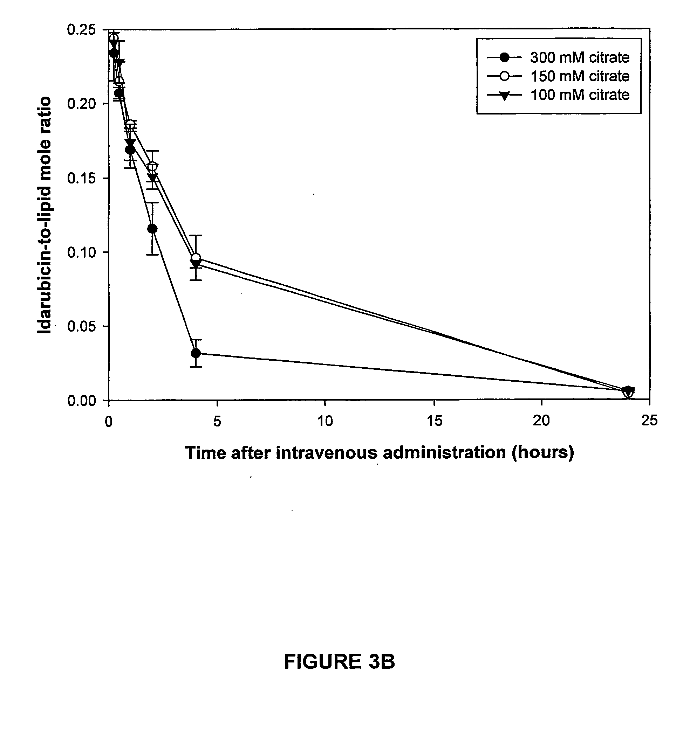

**Figure 3B:** A graph showing idarubicin-to-lipid mole ratio in the blood after intravenous injection of Balb/c mice at various time points for liposomes consisting of DSPC/DSPE-PEG2000 (95:5 mole ratio) utilizing 100 mM (filled triangles), 150 mM (open circles) and 300 mM (filled circles) citrate, pH 4 (200, 300 and 600 mOsm/kg, respectively) as the internal loading buffer.

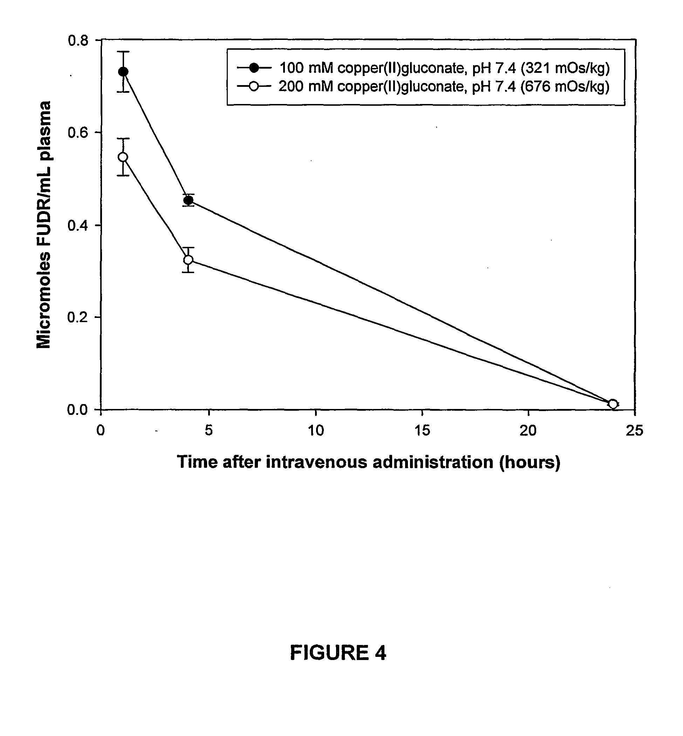

**Figure 4:** A graph showing Floxuridine (FUDR) levels remaining in the blood after intravenous administration of Balb/c mice with DSPC/DSPG/Chol (70:20:10 mole ratio) liposomes comprising copper(II)gluconate at the indicated osmolarities. Blood was collected at 1, 4 and 24-hours after intravenous injection.

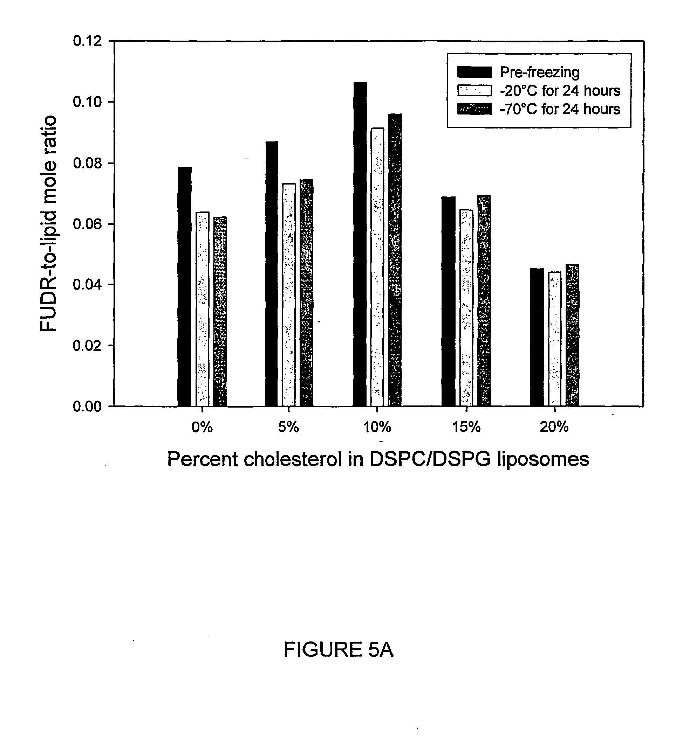

**Figure 5A:** A histogram showing the drug-to-lipid ratio of irinotecan prior to and after freezing of DSPC/DSPG liposomes (with 0-20 mole % cholesterol) comprising encapsulated irinotecan and FUDR and utilizing 250 mM $CuSO_4$ (< 500 mOsm/kg) as the intraliposomal solution. Freezing was performed for 24 hours at either -20°C or -70°C.

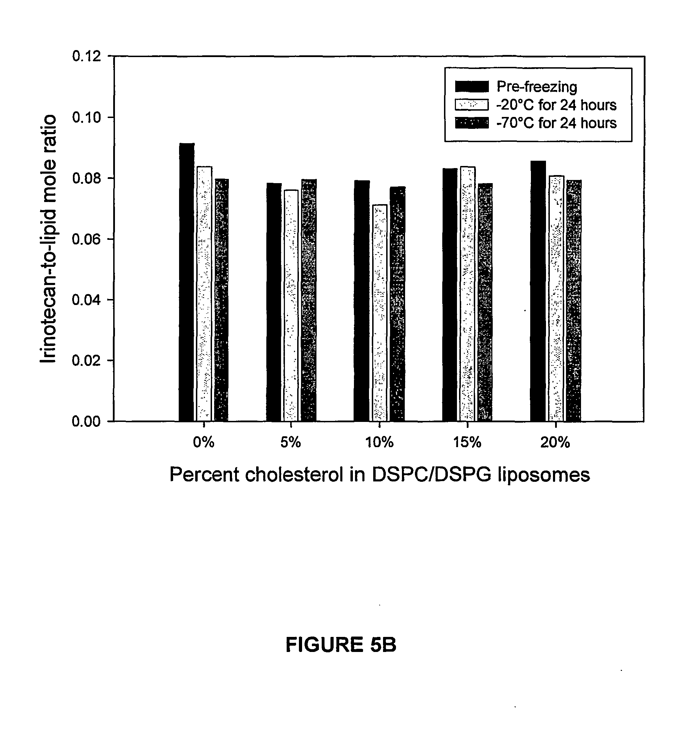

**Figure 5B:** A histogram showing the size of DSPC/DSPG liposomes (with 0-20 mole % cholesterol) comprising FUDR and irinotecan and utilizing 250 mM $CuSO_4$ as the intraliposomal solution prior to and after freezing. Freezing was performed for 24 hours at either -20°C or -70°C.

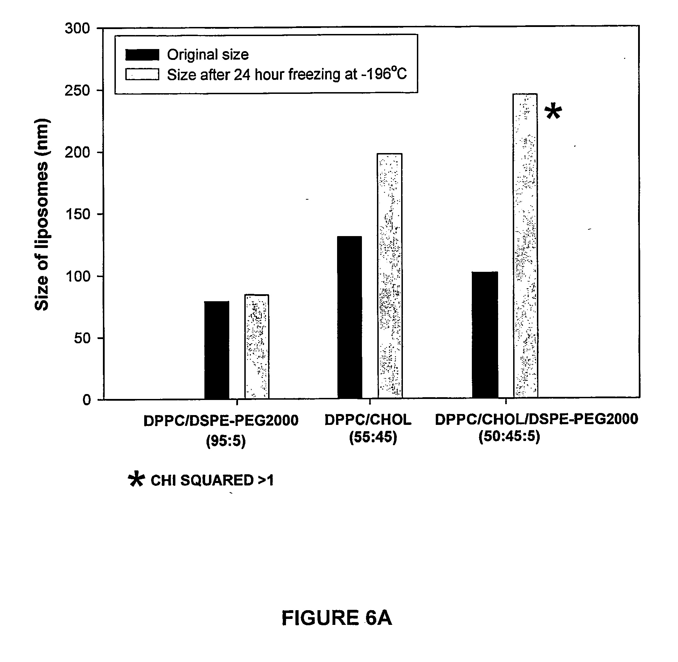

**Figure 6A:** A histogram showing the size of liposomes comprising HBS, pH 7.4 (20 mM HEPES, 150 mM NaCl; corresponding to approximately 320 mOsm/kg), both inside and outside the liposomal membrane prior to (black bar) and subsequent to (grey bar) freezing in liquid nitrogen for 24 hours. Liposomes consisting of DPPC/DSPE-PEG2000 (95:5 mole ratio), DPPC/cholesterol (55:45 mole ratio) and DPPC/cholesterol/DSPE-PEG2000 (50:45:5 mole ratio) were tested.

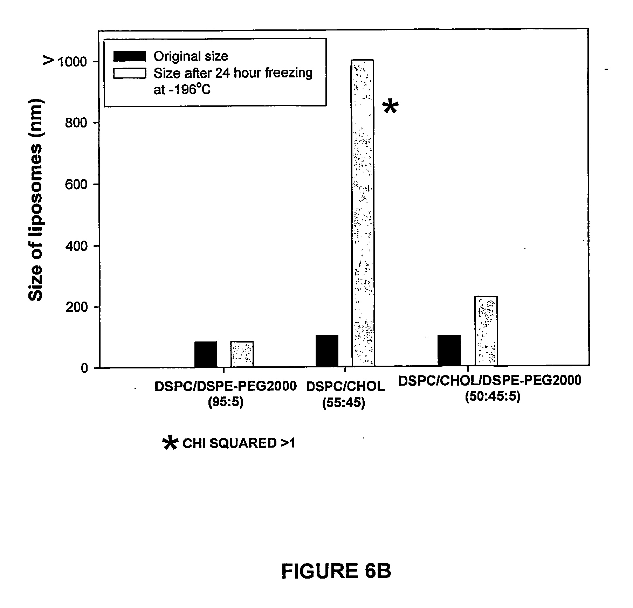

**Figure 6B:** A histogram showing the size of liposomes containing HBS, pH 7.4 (approximately 320 mOsm/kg) both inside and outside the liposomal membrane prior to (black bar) and subsequent to (grey bar) freezing in liquid nitrogen for 24 hours. Liposomes consisting of DSPG/DSPE-PEG2000 (95:5 mole ratio), DSPC/cholesterol (55:45 mole ratio) and DSPC/cholesterol/DSPE-PEG2000 (50:45:5 mole ratio) were tested.

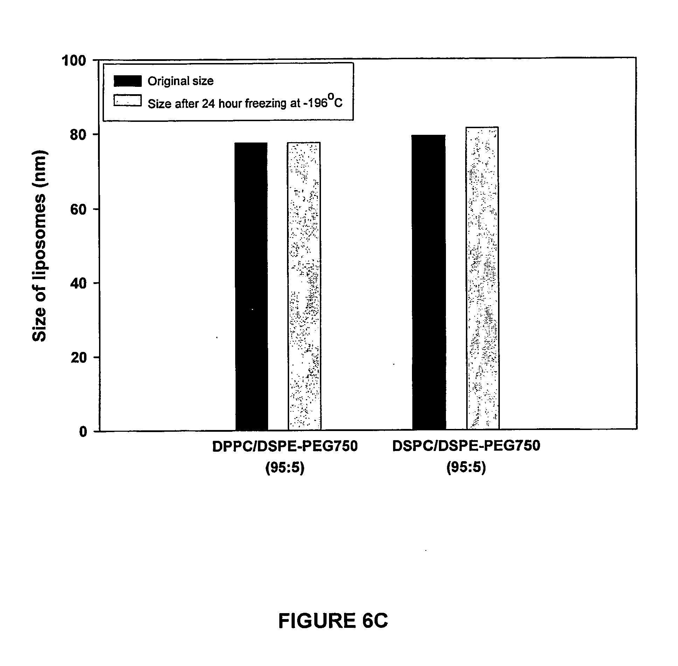

**Figure 6C:** A histogram showing the size of liposomes containing HBS, pH 7.4 (approximately 320 mOsm/kg) both inside and outside the liposomal membrane prior to (black bar) and subsequent to (grey bar) freezing in liquid nitrogen for 24 hours. Liposomes consisting of DPPC/DSPE-PEG750 (95:5 mole ratio) and DSPC/DSPE-PEG750 (95: 5 mole ratio) were tested.

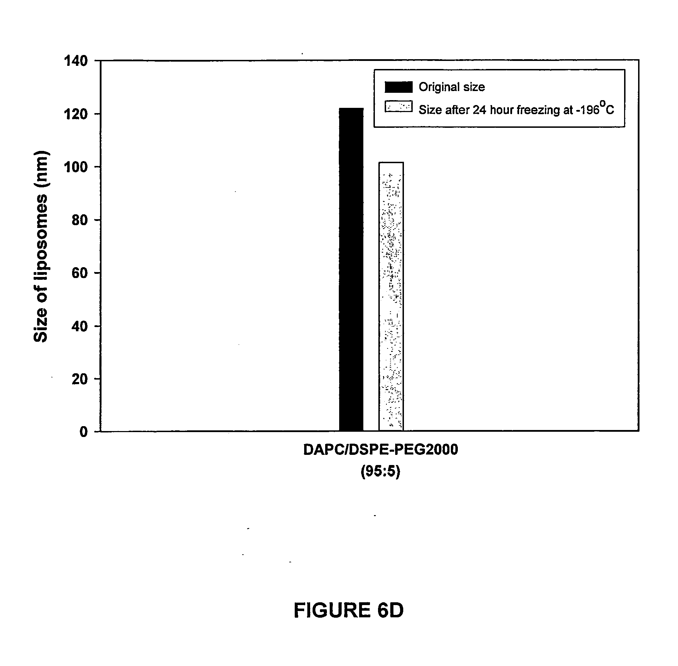

**Figure 6D:** A histogram showing the size of liposomes containing HBS, pH 7.4 (approximately 320 mOsm/kg) both inside and outside the liposomal membrane prior to (black bar) and subsequent to (grey bar) freezing in liquid nitrogen for 24 hours. Liposomes consisting of DAPC/DSPE-PEG2000 (95:5 mole ratio) were tested.

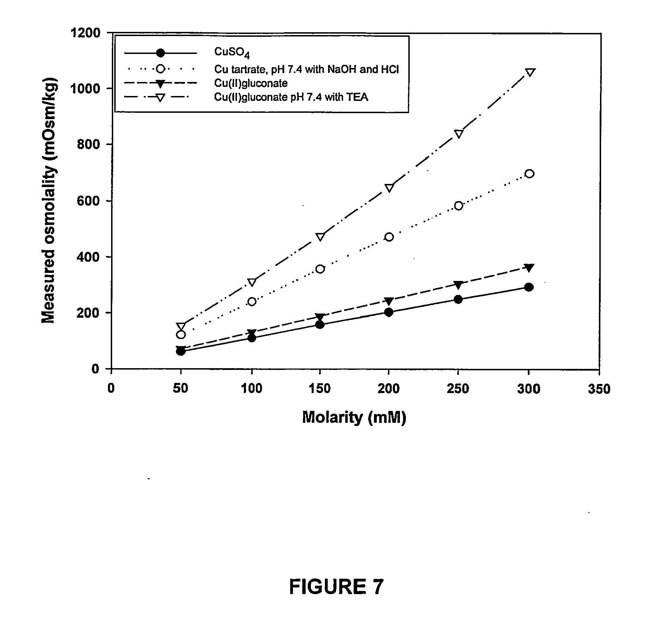

**Figure 7:** A graph showing the increase in measured osmolality (mOsm/kg) as a function of increasing concentrations (mM) of $CuSO_4$ (closed circles), copper tartrate pH adjusted to 7.4 with NaOH and HCl (open circles), copper gluconate (closed triangles) and copper gluconate pH adjusted to 7.4 with TEA (open triangles).

Modes of Carrying Out the Invention

The following abbreviations are used. PEG: polyethylene glycol; PEG preceded or followed by a number: the number is the molecular weight of PEG in Daltons; PEG-lipid: polyethylene glycol-lipid conjugate; PE-PEG: polyethylene glycol-derivatized phosphatidylethanolamine;

PA: phosphatidic acid;

PE: phosphatidylethanolamine;

PC: phosphatidylcholine;

PI: phosphatidylinositol;

DSPC: 1,2-distearoyl-*sn*-glycero-3-phosphocholine;

DSPE-PEG 2000 (or 2000 PEG-DSPE or $PEG_{2000}$-DSPE): 1,2-distearoyl-*sn*-glycero-3-phosphoethanolamine-N-[polyethylene glycol 2000];

DSPE-PEG 750 (or 750PEG-DSPE or $PEG_{750}$-DSPE): 1,2-distearoyl-*sn*-glycero-3-phosphoethanolamine-N-[polyethylene glycol 750];

DPPE-PEG2000: 1,2-dipalmaitoyl-*sn*-glycero-3-phosphoethanolamine-N-[polyethylene glycol 2000];

DAPC: 1,2-arachidoyl-*sn*-glycero-3-phosphocholine;

DBPC: 1,2-dibehenoyl-*sn*-glycero-3-phosphocholine;

CH or Chol: cholesterol;

DPPC: 1,2-dipalmaitoyl-*sn*-glycero-3-phosphocholine;

HEPES: N-[2-hydroxylethyl]-piperazine-N-[2-ethanesulfonic acid].

"Substantially no cholesterol" with reference to a liposome means that a liposome is prepared in the absence of, and contains no cholesterol, or that the liposome contains only an amount of cholesterol that is insufficient to significantly alter the phase transition characteristics of the liposome, *i.e.*, typically less than 20 mol % cholesterol; 20 mol % or more of cholesterol broadens the range of temperatures at which phase transition occurs, with phase transition disappearing at higher cholesterol levels. Preferably, a liposome having substantially no cholesterol will have less than 15 mol % and more preferably less than 10 mol % cholesterol, more preferably less than 5 mol %, or less than 2 mol % and even less than 1 mol % cholesterol. Most preferably, no cholesterol will be present or added. Cholesterol free and substantially cholesterol free liposomes are described in co-pending international patent application PCT/CA01/00655.

The term "liposome" as used herein means vesicles comprised of one or more concentrically ordered lipid bilayers encapsulating an aqueous phase. Included in this definition are unilamellar vesicles. The term "unilamellar vesicle" as used herein means single-bilayer vesicles or substantially single-bilayer vesicles encapsulating an aqueous phase wherein the vesicle is less than 500 nm. The unilamellar vesicle is preferably a "large unilamellar vesicle (LUV)" which is a unilamellar vesicle between 500 and 50 nm, preferably 200 to 80 nm.

Formation of liposomes requires the presence of "vesicle-forming lipids" which are amphipathic lipids capable of either forming or being incorporated into a bilayer structure. The latter term includes lipids that are capable of forming a bilayer by themselves or when in combination with another lipid or lipids. An amphipathic lipid is incorporated into a lipid bilayer by having its hydrophobic moiety in contact with the interior, hydrophobic region of the membrane bilayer and its polar head moiety oriented toward an outer, polar surface of the membrane. Hydrophilicity may arise from the presence of functional groups such as hydroxyl, phosphato, carboxyl, sulfato, amino or sulfhydryl groups. Hydrophobicity results from the presence of a long chain of alaphatic hydrocarbon groups. The vesicle forming lipids included in the liposomes of the invention will typically comprise at least one acyl group with a chain length of at least 16 carbon atoms. Thus, for example, preferred phospholipids used as vesicle forming components include dipalmitoyl phosphatidylcholine (DPPC) and distearol phosphatidylcholine (DSPC).

The liposomes of the invention comprise amphipathic lipids as vesicle forming lipids, but no substantial amount of cholesterol. Such lipids include sphingomyelins, glycolipids, ceramides and phospholipids. Such lipids may include lipids having targeting agents, ligands, antibodies or other such components which are used in liposomes, either covalently or non-covalently bound to lipid components.

The liposomes of the invention will also contain, in one embodiment, an effective amount of one or more components that prevent aggregation and surface-surface interactions ("aggregation preventing agents") such as phosphatidyl glycerol (PG), phosphatidyl inositol (PI) and/or a modified lipid containing a hydrophilic polymer, such as PEG. These components are typically present at 1-30 mol % of the lipid bilayer, or 3-15 mol % or 5-10 mol % or 10-30 mol %. They are present in an effective amount to maintain the integrity of the individual liposomes in the composition. It will be noted that some of these components may, in themselves, be vesicle forming lipids; some vesicle forming lipids as defined above may also provide the aggregation prevention activities desired. There is no bright line between lipids which are "vesicle forming" and those which are "aggregation preventing."

The liposomes of the invention are characterized by an internal aqueous medium that has an osmolarity of 500 mOsm/kg or less, or 200 mOsm or less or 300 mOsm or less. The osmolarity can be measured using standard laboratory devices designed to measure colligative properties such as a freezing point osmometer. Colligative properties are determined by the number of particles in solution, so that for ionized substances, the osmolarity will be determined by the concentration of individual ions present in solution. It is understood that theoretical calculations of such ion concentrations must be modified by a factor to correct for incomplete ionization and/or differences in activity coefficient. For clarity, as used in the present case, an osmolarity is defined as the intraliposomal osmolarity as calculated and determined in a manner described hereinbelow. As set forth in the examples below, various techniques have been described for such determinations, including those established by Perkins, et al., Biochim. Biophys. Acta (1988) 943:103-107. As set forth above, a rough estimate of the osmolarity can be determined from the concentration of individual ions, especially in dilute solutions. However, the estimate will not be precise due to the factors mentioned above.

It is important that the intraliposomal osmolarity be measured since certain ions, as they readily cross the bilipid layer, appear not to affect the osmolarity of the internal aqueous medium. For example, solutions of sodium chloride, while they may be included in the initial preparations, appear not to affect the osmolarity of the internal medium due to the property of chloride ions readily to cross this barrier.

Liposomes of the present invention or for use in the present invention may be generated by a variety of techniques including but not limited to lipid film/hydration, reverse phase evaporation, detergent dialysis, freeze/thaw, homogenation, solvent dilution and extrusion procedures. Preferably, the liposomes are generated by extrusion procedures as described by Hope, et al., Biochim. Biophys. Acta (1984) 55-64 and set forth in the Examples below.

Liposomes of the invention contain an encapsulated biologically active agent. These agents are typically small molecule drugs useful in treatment of neoplasms or may be antibiotics. Suitable drugs, for example, include cisplatin, carboplatin, doxorubicin, gentamicin, and the like. The drugs are incorporated into the aqueous internal compartment (s) of the liposomes either by passive or active loading procedures. In passive loading, the biologically active agent is simply included in the preparation from which the liposomes are formed. Optionally, unencapsulated material may be removed from the preparation by known procedures. Alternatively, active loading procedures can be employed, such as ion gradients, ionophores, pH gradients and metal-based loading procedures based on metal complexation. One embodiment commonly employed for suitable drugs is loading via pH gradient.

Preferably, the biologically active agent is a drug and most preferably an antineoplastic agent. Examples of some of the antineoplastic agents which can be loaded into liposomes by this method and therefore may be used in this invention include but are not limited to anthracyclines such as doxorubicin, daunorubicin, mitoxanthrone, idarubicin, epirubicin and aclarubicin; antineoplastic antibiotics such as mitomycin and bleomycin; vinca alkaloids such as vinblastine, vincristine and vinorelbine; alkylating agents such as cyclophosphamide and mechlorethamine hydrochloride; campthothecins such as topotecan, ironotecan, lurtotecan, 9-aminocamptothecin, 9-nitrocamptothecin and 10-hydroxycamptothecin; purine and pyrimidine derivatives such as 5-fluorouracil; cytarabines such as cytosine arabinoside. This invention is not to be limited to those drugs currently available, but extends to others not yet developed or commercially available, and which can be loaded using the transmembrane pH gradients.

According to this technique, liposomes are formed which encapsulate an aqueous phase of a selected pH. Hydrated liposomes are placed in an aqueous environment of a different pH selected to remove or minimize a charge on the drug or other agent to be encapsulated. Once the drug moves inside the liposome, the pH of the interior results in a charged drug state, which prevents the drug from permeating the lipid bilayer, thereby entrapping the drug in the liposome.

To create a pH gradient, the original external medium is replaced by a new external medium having a different concentration of protons. The replacement of the external medium can be accomplished by various techniques, such as, by passing the lipid vesicle preparation through a gel filtration column, *e.g.*, a Sephadex column, which has been equilibrated with the new medium (as set forth in the examples below), or by centrifugation, dialysis, or related techniques. The internal medium may be either acidic or basic with respect to the external medium. A pH gradient may also be created by adjusting the pH of the external medium with a strong acid or base.

After establishment of a pH gradient, a pH gradient loadable agent is added to the mixture and encapsulation of the agent in the liposome occurs as described above. Preferably the ratio of the agent to the lipid making up the liposome is less than 0.4.

The term "pH gradient loadable agent" refers to agents with one or more ionizable moieties such that the neutral form of the ionizable moiety allows the drug to cross the liposome membrane and conversion of the moiety to a charged form causes the drug to remain encapsulated within the liposome. The biologically active agent may be a drug, a diagnostic agent, or a nutritional supplement. Ionizable moieties may comprise, but are not limited to comprising, amine, carboxylic acid and hydroxyl groups. pH gradient loadable agents that load in response to an acidic interior may comprise ionizable moieties that are charged in response to an acidic environment whereas drugs that load in response to a basic interior comprise moieties that are charged in response to a basic environment. In the case of a basic interior, ionizable moieties including but not limited to carboxylic acid or hydroxyl groups may be utilized. In the case of an acidic interior, ionizable moieties including but not limited to primary, secondary and tertiary amine groups may be used.

The term "internal loading buffer" includes a buffer encapsulated in the interior of a liposome which facilitates pH gradient loading and retention of a pH gradient loadable drug in a liposome after intravenous administration. The combined osmolarity of all internal loading buffers present in the interior of the liposome does not exceed 500 mOsm/kg.

In general, internal buffer solutions useful in embodiments of the present invention are chosen so that the pharmaceutical agent to be accumulated has a solubility within the internal buffer solution which is less than the total agent to be accumulated in the liposome.

Where the pH gradient loadable drug is one that loads in response to a transmembrane pH gradient wherein the inside of the liposome is relatively basic with respect to the outside, an internal loading buffer such as, but not limited to, sodium carbonate may be used in conjunction with an exterior buffer such as potassium sulfate/ HEPES buffer (interior buffer/exterior buffer). Internal buffers are best used at a pH of about 6.0 to 11.0 and external buffers are best used at a pH of 6.5 to 8.5.

Where the pH gradient loadable drug is one that loads in response to a transmembrane pH gradient where the interior of the liposome is relatively acidic with respect to the exterior, acidic internal loading buffers may be used. The acidic loading buffers, which in general can be used in practicing this invention include organic acids, *e.g.*, monofunctional pyranosidyl acids such as glucuronic acid, gulonic acid, gluconic acid, galacturonic acid, glucoheptonic acid, lactobionic acid, and the like, alpha-hydroxy polycarboxylic acids such as citric acid, iso-citric acid, hyaluronic acid, carboxypolymethylenes, and the like, amino acids such as aspartic acid, carboxyaspartic acid, carboxyglutamic acid, and the like, saturated and unsaturated, unsubstituted and substituted aliphatic dicarboxylic acids such as succinic acid, glutaric acid, ketoglutaric acid, tartaric acid, galactaric acid, maleic acid, fumaric acid, glucaric acid, malonic acid, and the like, phosphorus-containing organic acids such as phytic acid, glucose phosphate, ribose phosphate, and the like, and inorganic acids, *e.g.*, sulfonic acid, sulfuric acid, phosphoric acid, polyphosphoric acids, and the like. Such buffers are best used at pH of about 2.0 to 4.5. Preferably, the interior buffer is an $\alpha$-hydroxy polycarboxylic acid such as citric acid. The exterior buffer may be a buffer present at neutral pH such as HEPES, pH 7.0. Most preferably, the internal buffer is citrate, pH 2.0 to 4.0. The internal buffer osmolarity of the liposome is less than 500 mOsm/kg, preferably less than 300 mOsm/kg.

Additional internal buffers that may be used in this invention are those which comprise an ionizable moiety that is neutral when deprotonated and charged when protonated. The neutral deprotonated form of the buffer (which is in equilibrium with the protonated form) is able to cross the liposome membrane and thus leave a proton behind in the interior of the liposome and thereby cause an increase in the pH of the interior. Examples of such buffers include methylammonium chloride, methylammonium sulfate, ethylenediammonium sulfate and ammonium sulfate. Internal loading buffers that are able to establish a basic internal pH, can also be utilized. In this case, the neutral form of the buffer is protonated such that protons are shuttled out of the liposome interior to establish a basic interior. An example of such a buffer is calcium acetate.

Liposomes of the present invention may be prepared such that they are sensitive to elevations of the temperature in the surrounding environment. The temperature-sensitivity of such liposomes allows the release of compounds entrapped within the interior aqueous space of the liposome, and/or the release of compounds associated with the lipid bilayer, at a target site that is either heated (as in the clinical procedure of hyperthermia) or that is at an intrinsically higher temperature than the rest of the body (as in inflammation). Liposomes that allow release of compounds in a temperature dependent manner are termed "thermosensitive liposomes." The liposomes may comprise a lipid possessing a gel-to-liquid crystalline transition temperature in the hyperthermic range (*e.g.,* the range of from approximately 38°C to approximately 45°C). Preferred are phospholipids with a phase-transition temperature of from about 38°C to about 45°C. A particularly preferred phospholipid is dipalmitoylphosphatidylcholine (DPPC). DPPC is a common saturated chain (C 16) phospholipid with a bilayer transition of 41.5°C. Thermosensitive liposomes containing DPPC and other lipids that have a similar or higher transition temperature, and that mix ideally with DPPC (such 1,2-dipalmitoyl-sn-glycero-3-[phospho-rac-(1-glycerol)] (DPPG) (Tc=41.5°C) and 1,2-distearoyl-sn-glycero-3-phosphocholine (DSPC) (Tc=55.1°C)) have been studied.

Thus, the liposomes of the invention typically have transitioned temperature greater than 38°C; this can be assured by employing components which confer this property. Among diacyl phosphatidyl glycerides, typically the acyl chains contain at least 16 carbons. However, the ultimate transition temperature will also depend on the degree of unsaturation of the acyl groups. Typically, including unsaturation in the chain lowers the transition temperature so that in the event the acyl groups are unsaturated, acyl groups containing 18 carbons or 20 carbons or more are preferred.

Thermosensitive liposomes of the present invention may incorporate a relatively-water soluble surface active agent, such as a lysolipid, into a bilayer composed primarily of a relatively water-insoluble molecule, such as a di-chain phospholipid (*e.g.*, DPPC). Incorporation of the surface active agent in the gel phase of the primary lipid component enhances the release of contents from the resulting liposome when heated to the gel-liquid crystalline phase transition temperature of the primary lipid. Preferred surface active agents are lysolipids, and a particularly preferred surface active agent is monopalmitoylphosphatidylcholine (MPPC). Suitable surface-active agents are those that are compatible with the primary lipid of the bilayer, and that desorb when the lipid melts to the liquid phase. Additional suitable surface-active agents for use in phospholipid bilayers include palmitoyl alcohols, stearoyl alcohols, palmitoyl, stearoyl, glyceryl monopalmitate, glyceryl monooleate, and mono-acylated lipids such as sphingosine and sphingamine.

Liposomes may also be prepared such that the liquid crystalline transition temperature is greater than 45°C. Vesicle-forming lipids making up the liposome are phospholipids such as phosphatidylcholine (PC), phosphatidylethanolamine (PE), phosphatidyl (PA) or phosphatidylethanolamine (PE), containing two saturated fatty acids, within the acyl chains are preferably stearoyl (18:0), nonadecanoyl (19:0), arachidoyl (20:0), heniecosanoyl (21:0), behenoyl (22:0), tricosanoyl (23:0), lingnoceroyl (24:0) or cerotoyl (26:0).

Grafting a hydrophilic polymer such as a polyalkylether to the surface of liposomes has been utilized to sterically stabilize liposomes to minimize protein adsorption to liposomes. This results in enhanced blood stability and increased circulation time, reduced uptake into healthy tissues, and increased delivery to disease sites such as solid tumors. United States patents 5,013,556 and 5,593,622. These moieties are "aggregation preventing agents." Typically, the polymer is conjugated to a lipid component of the liposome. A preferred hydrophilic polymer is polyethylene glycol (PEG). This "hydrophilic polymer-lipid conjugate" is an example of an aggregation preventing agent where a vesicle-forming lipid is covalently joined at its polar head moiety to a hydrophilic polymer. It is typically made from a lipid that has a reactive functional group at the polar head moiety in order to attach the polymer. Suitable reactive functional groups are for example, amino, hydroxyl, carboxyl or formyl groups. The lipid may be any lipid described in the art for use in such conjugates other than cholesterol. Preferably, the lipid is a phospholipid such as acylated PC, PE, PA or PI, having two acyl chains comprising between about 6 to about 24 carbon atoms in length with varying degrees of unsaturation. For example, the lipid in the conjugate may be a PE, preferably of the distearoyl form. The polymer is a biocompatible polymer characterized by a solubility in water that permits polymer chains to effectively extend away from a liposome surface with sufficient flexibility that produces uniform surface coverage of a liposome. Preferably, the polymer is a polyalkylether, including polymethylene glycol, polyhydroxy propylene glycol, polypropylene glycol, polylactic acid, polyglycolic acid, polyacrylic acid and copolymers thereof, as well as those disclosed in United States patents 5,013,556 and 5,395,619. A preferred polymer is polyethylene glycol (PEG). Preferably, the polymer has a molecular weight between about 1000 and 5000 daltons; however, polymers of less than 1000 daltons such as 750, 500 and 350 have also been shown to effectively extend the circulation lifetime of cholesterol free liposomes. The conjugate may be prepared to include a releasable lipid-polymer linkage such as a peptide, ester, or disulfide linkage. The conjugate may also include a targeting ligand. Mixtures of conjugates may be incorporated into liposomes for use in this invention.

The term "PEG-conjugated lipid" as used herein refers to the above-defined hydrophilic polymer-lipid conjugate in which the polymer is PEG.

The liposomes of the invention may include one or more "reactive phospholipids" *i.e.,* a phospholipid in which the glyceryl phosphate group is coupled to an α-amino acid, covalently joined to the side chain of the α-amino acid. Included in this class are the phosphoglycerides such as phosphatidylserine (PS) and the sphingolipids which have two hydrocarbon chains in the hydrophobic portion that are between 5-23 carbon atoms in length and have varying degrees of saturation. The amino acid may be natural or synthetic and of the D or L configurations. Preferably the side chain of the amino acid is a straight or branched alkyl group having between 1 and 3 carbons, including saturated, mono and disubstituted alkyls. Preferably the reactive phospholipid is a phosphotriglyceride wherein the hydrophobic portion results from the esterification of two C6-C24 fatty acid chains with the hydroxyl groups at the 1- and 2- positions of glycerol, where the two fatty acid chains are independently caproyl (6:0), octanoyl (8:0), capryl (10:0), lauroyl (12:0), mirystoyl (14:0), palmitoyl (16:0), stearoyl (18:0), arachidoyl (20:0), behenoyl (22:0), lingnoceroyl (24:0) or phytanoyl, including the unsaturated versions of these fatty acid chains in the cis or trans configurations such as oleoyl (18:1), linoleoyl (18:2), erucoyl (20:4) and docosahexaenoyl (22:6).

The liposomes of the present invention may be administered to warm-blooded animals, including humans. These liposome and lipid carrier compositions may be used to treat a variety of diseases in warm-blooded animals. Examples of medical uses of the compositions of the present invention include but are not limited to treating cancer, treating cardiovascular diseases such as hypertension, cardiac arrhythmia and restenosis, treating bacterial, fungal or parasitic infections, treating and/or preventing diseases through the use of the compositions of the present inventions as vaccines, treating inflammation or treating autoimmune diseases. For treatment of human ailments, a qualified physician will determine how the compositions of the present invention should be utilized with respect to dose, schedule and route of administration using established protocols. Such applications may also utilize dose escalation should bioactive agents encapsulated in liposomes and lipid carriers of the present invention exhibit reduced toxicity to healthy tissues of the subject.

Pharmaceutical compositions comprising the liposomes of the invention are prepared according to standard techniques and further comprise a pharmaceutically acceptable carrier. Generally, normal saline will be employed as the pharmaceutically acceptable carrier. Other suitable carriers include, *e.g.*, water, buffered water, 0.4% saline, 0.3% glycine, and the like, including glycoproteins for enhanced stability, such as albumin, lipoprotein, globulin, etc. These compositions may be sterilized by conventional, well known sterilization techniques. The resulting aqueous solutions may be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, etc. Additionally, the liposome suspension may include lipid-protective agents which protect lipids against free-radical and lipid-peroxidative damages on storage. Lipophilic free-radical quenchers, such as alphatocopherol and watersoluble iron-specific chelators, such as ferrioxamine, are suitable.

The concentration of liposomes, in the pharmaceutical formulations can vary widely, *i.e.,* from less than about 0.05%, usually at or at least about 2-5% to as much as 10 to 30% by weight and will be selected primarily by fluid volumes, viscosities, etc., in accordance with the particular mode of administration selected. For example, the concentration may be increased to lower the fluid load associated with treatment. Alternatively, liposomes composed of irritating lipids may be diluted to low concentrations to lessen inflammation at the site of administration. For diagnosis, the amount of liposomes administered will depend upon the particular label used, the disease state being diagnosed and the judgement of the clinician but will generally be between about 0.01 and about 50 mg per kilogram of body weight, preferably between about 0.1 and about 5 mg/kg of body weight.

Preferably, the pharmaceutical compositions are administered intravenously. Typically, the formulations will comprise a solution of the liposomes suspended in an acceptable carrier, preferably an aqueous carrier. A variety of aqueous carriers may be used, *e.g.*, water, buffered water, 0.9% isotonic saline, 5 % dextrose and the like. These compositions may be sterilized by conventional, well known sterilization techniques, or may be sterile filtered. The resulting aqueous solutions may be packaged for use as is, or lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

Dosage for the liposome formulations will depend on the ratio of drug to lipid and the administrating physician's opinion based on age, weight, and condition of the patient.

The methods of the present invention may be practiced in a variety of hosts. Preferred hosts include mammalian species, such as humans, non-human primates, dogs, cats, cattle, horses, sheep, and the like.

The present invention is further described by the following examples. The examples are provided solely to illustrate the invention by reference to specific embodiments.

Example 1

Optimal retention of vincristine in low-cholesterol liposomes is achieved utilizing an internal osmolarity of less than 500 mOsm/kg

The effect of intraliposomal osmolarity on the retention of drug in cholesterol-free and cholesterol-containing liposomes was investigated using 1,2-distearoyl-*sn*-glycero-3-phosphocholine (DSPC) / 1,2-distearoyl-*sn*-glycero-3 phosphoethanolannine-N-[polyethylene glycol 2000] (DSPE-PEG2000) and DSPC/Cholesterol liposomes with encapsulated vincristine.

Solutions of lipids in chloroform were combined to give a 95:5 molar ratio of DSPC/DSPE-PEG2000 or a 55: 45 molar ratio of DSPC/Cholesterol, with trace amounts of $^{14}$C-cholesteryl hexadecyl ether ($^{14}$C-CHE). The resulting mixtures were dried under a stream of nitrogen gas and placed in a vacuum pump overnight. The samples were hydrated at 70°C with either 300 mM citrate, pH 4.0 (about 600 milliosmoles/kg (mOsm/kg)) or 150 mM citrate buffer, pH 4.0 (about 300 mOsm/kg) and passed through an extrusion apparatus (Northern Lipids Inc., Vancouver, BC) ten times with two 100 nm pore size polycarbonate filters at 70°C. Average liposome size was determined by quasi-elastic light scattering using a NICOMP 370 submicron particle sizer operating at a wavelength of 632.8 nm. The resulting liposomes were applied to a Sephadex G50 column equilibrated with HBS (20 mM HEPES, 150 mM NaCl, about 320 mOsm/kg), pH 7.45 to exchange the external liposomal buffer. Liposomes were subsequently combined with vincristine (and trace amounts of radiolabeled vincristine) at a 0.1:1 drug to lipid weight ratio. To facilitate drug loading, the mixtures were first incubated at 37°C for ten minutes.

Vincristine-containing liposomes were administered intravenously to Balb/c mice at a lipid dose of 165 $\mu$moles/kg in a final volume of 200 $\mu$L immediately after preparation (within 1-2 hrs). Blood samples were removed by cardiac puncture at 1, 4 and 24-hours post administration (3 mice per time point). Lipid and vincristine levels were quantified by liquid scintillation counting and the values were reported as the mean $\pm$ standard deviation (SD).

Figure 1 shows that retention of vincristine in low-cholesterol liposomes is significantly enhanced when citrate at an osmolarity of 300 mOsm/kg (150 mM; open circles) is utilized as the internal loading buffer compared to 600 mOsm/kg (300 mM; closed circles). Retention of vincristine in cholesterol containing liposomes is independent of the osmolarity of the intraliposomal solution.

Example 2

Daunorubicin is optimally retained in low-cholesterol liposomes utilizing internal buffers of low osmolarity

To further investigate the effect of internal osmolarity on drug retention in low-cholesterol liposomes, daunorubicin was also loaded into DSPC/DSPE-PEG2000 liposomes comprising citrate of either high or low osmolarity. The *in vivo* retention of daunorubicin was also determined in DSPC/Cholesterol and DSPC/Cholesterol/DSPE-PEG2000 liposomes prepared with an internal citrate concentration of low osmolarity.

DSPC/DSPE-PEG2000 liposomes (95:5 mole ratio) containing 150 or 300 mM citrate (300 or 600 mOsm/kg), pH 4 and DSPC/Cholesterol (55:45 mole ratio) and DSPC/Cholesterol/DSPE-PEG2000 (50:45:5 mole ratio) liposomes containing 150 mM citrate, pH 4 were prepared as described in Example 1. Liposomes were subsequently combined with daunorubicin at a 0.2:1 drug to lipid mole ratio. To facilitate drug loading, the mixtures were incubated at 40°C for 60 minutes.

The resulting daunorubicin-containing liposomes were administered to Balb/c female mice at a lipid dose of 165 $\mu$moles/kg as detailed above. Blood samples were removed at 1, 4 and 24 hours post administration, by cardiac puncture (3 mice per time point). Lipid levels were determined by liquid scintillation counting. Daunorubicin was extracted from plasma samples and quantified as follows: a defined volume of plasma was adjusted to 200 $\mu$L with distilled water followed by addition of 600 $\mu$L of distilled water, 100 $\mu$L of 10% SDS and 100 $\mu$L of 10 mM $H_2SO_4$. This solution was mixed and 2 mLs of 1:1 isopropanol/chloroform was added followed by vortexing. The samples were frozen at -20°C overnight or -80°C for 1 hour to promote protein aggregation, brought to room temperature, vortexed and centrifuged at 3000 rpm for 10 minutes. The bottom organic layer was removed and assayed for fluorescence intensity at 500 nm as the excitation wavelength (2.5 nm bandpass) and 550 nm as an emission wavelength (10 nm bandpass) and using an absorbance wavelength of 480 nm.

Figure 2 shows that, like vincristine (Figure 1), low-cholesterol liposomes prepared with citrate at 600 mOsm/kg (300 mM; open triangles) as the internal buffer displayed compromised daunorubicin retention in relation to low-cholesterol liposomes with an internal buffer osmolarity of 300 mOsm/kg (150 mM; closed triangles). All values are reported as the mean $\pm$ SD.

Example 3

Liposomes with decreasing intraliposomal osmolarites display enhanced retention of drug

In order to examine the effect of decreasing internal osmolarity on drug retention in low-cholesterol liposomes, daunorubicin and idarubicin were loaded into DSPC/DSPE-PEG2000 liposomes containing varying amounts of citrate.
DSPC/DSPE-PEG2000 (95:5 mole ratio) liposomes containing the non-exchangeable marker $^3$H-CHE were prepared as described in Example 1, except that lipid films were hydrated with 100, 150, 200, 250 or 300 mM citrate, pH 4.0 (corresponding to osmolarity levels of about 200, 300, 400, 500 or 600 mOsm/kg, respectively).
Daunorubicin was loaded at a 0.2:1 drug-to-lipid mole ratio with the methods detailed above into each of the five liposomal formulations. The resulting liposomes were administered to female Balb/c mice at a lipid dose of 165 $\mu$moles/kg in a final volume of 200 $\mu$L immediately after preparation (within 1-2 hrs). Blood samples were removed 4 hours after administration by cardiac puncture (3 mice per time point). Lipid and daunorubicin levels were determined as described previously and percent initial drug-to-lipid ratio was reported as the mean $\pm$ SD.
Figure 3A shows that low cholesterol containing liposomes utilizing 100, 150 and 200 mM internal buffer concentrations with osmolarities of about 200, 300 and 400 mOsm/kg, respectively, exhibit optimal retention of daunorubicin in low-cholesterol liposomes.
DSPC/DSPE-PEG2000 (95:5 mole ratio) liposomes prepared with 100, 150 and 300 mM citrate were also loaded with idarubicin at a drug to lipid mole ratio of 0.25:1. Loading was facilitated by incubating the drug and liposomes at 37°C for 60 minutes. Liposomes were administered to Balb/c mice as indicated and blood samples were removed by cardiac puncture at 0.5, 1, 2, 4 and 24-hours post administration (3 mice per time point). Idarubicin concentration was quantitated using fluorescence intensity at 485 nm as the excitation wavelength and 535 nm as an emission wavelength and using an absorbance wavelength of 482 nm.
Figure 3B illustrates that liposomes prepared in the absence of cholesterol and having an internal osmolarity of greater than 500 mOsm/kg (300 mM citrate; closed circles) displayed significantly decreased idarubicin retention in relation to cholesterol-free liposomes with intraliposomal osmolarities of less than 500 mOsm/kg (100 and 150 mM).

Example 4

Improved retention of Floxuridine (FUDR) in low-osmolarity liposomes comprising phosphatidylglyercol as the stabilizing lipid

It is well documented that liposomes prepared with hydrophilic polymers such as polyethylene glycol (PEG) and lipids such as $GM_1$ have the ability to extend the circulation lifetime of liposomes. Studies in the preceding examples have made use of PEG's ability to stabilize, or reduce aggregation, of low-cholesterol and low-osmolarity liposomes. In order to investigate the effect of using phosphatidylglycerol (PG) as a stabilizing agent for low-cholesterol liposomes comprising intraliposomal solutions of low osmolarity, liposomes comprising distearoylphosphatidylglycerol (DSPG) and various internal osmolarities were tested for their retention of FUDR over a 24-hour time course.
DSPC/DSPG/Chol (70:20:10 mole ratio) were prepared following the methods of Example 1 except that lipid films were hydrated in either saline or Cu(II)gluconate, pH 7.4 containing 25 mg/mL FUDR at 70°C. Cu(II)gluconate was added at either 100 or 200 mM (321 and 676 mOsm/kg, respectively) and the pH was adjusted to 7.4 by addition of triethanolamine (TEA). Trace amounts of $^{14}$C-CHE and $^3$H-FUDR were used as lipid and drug markers, respectively. The resulting MLVs were extruded at 75°C through two stacked 100 nm pore size filters for a total of ten passes. Liposomes were buffer exchanged into HBS, pH 7.4 using a hand-held tangential flow column. A total lipid dose of 3.3 $\mu$moles (165 $\mu$moles/kg) was administered to female Balb/c mice in a final volume of 200 $\mu$L immediately after preparation (within 1-2 hrs). Blood samples were removed by cardiac puncture 1, 4 and 24-hours post administration (3 mice per time point). Lipid and FUDR levels were determined using liquid scintillation counting and values were reported as the mean $\pm$ SD.
The graph in Figure 4 shows that FUDR is optimally retained in cholesterol-deficient liposomes wherein the intraliposomal solution has an osmolarity of less than 500 mOsm/kg. These results thus demonstrate that the polymer, poly(ethylene glycol) (PEG), is not required and that non-zwitterionic moieties such as glycerol attached to the head group provide the same stabilizing function for these liposomes. Both PE lipid attached to PEG and the PG lipid contain a negatively charged phosphate group shielded by a hydrophilic neutral moiety. The presence of the hydroxy groups on the PG head group may facilitate hydrogen bonding with water molecules in the external medium creating a hydration shell surrounding the liposome. This would be in contrast to phosphatidylserine which has a negative and a positive charge at the terminus of the hydrophilic portion of the lipid due to the presence of a carboxylic acid group and an amine group respectively.

Example 5

Low-cholesterol PG-liposomes containing < 500 mOsm/kg internal solutions can be effectively frozen and thawed

It is preferable that liposome preparations exhibit extended chemical and physical stability properties in order for these compositions to be of practical use. This often requires the use of frozen or freeze-dried (lyophilized) product formats in order to avoid breakdown of labile drug and/or lipid components. However, when liposomes are frozen, ice crystal formation leads to mechanical rupture, liposome aggregation and fusion (measured by increases in liposome size subsequent to freezing) during the thawing/rehydration process as well as release of drugs that were entrapped inside the liposomes prior to freezing. These detrimental effects of freezing limit the commercial use of liposomes.

The following experiments demonstrate that liposomes of the present invention are resistant to fusion and leakage of agent subsequent to freezing:

FUDR and irinotecan were loaded into cholesterol-free liposomes containing a low osmolarity internal solution and drug retention and liposome size were measured prior to and after freezing. DSPC/DSPG liposomes containing 0-20 mole % cholesterol, 20 mole % DSPG and passively entrapped FUDR were prepared as described previously and hydrated in 250 mM $CuSO_4$ (< 500 mOsm/kg). The MLVs were extruded at 70°C as detailed above and buffered exchanged into saline and then into 300 mM sucrose, 20 mM Hepes, 30 mM EDTA (SHE), pH 7.4 using a hand-held tangential flow column. The sample was then further exchanged into 300 mM sucrose, 20 mM Hepes, pH 7.4 to remove residual EDTA. The liposomes were subsequently loaded with irinotecan at a drug-to-lipid mole ratio of 0.1:1 by mixing the two solutions at 50°C for five minutes. The dual-loaded liposomes were then buffered exchanged into HBS using tangential flow to remove any unencapsulated drug.

The influence of freezing on liposome stability was determined by freezing the liposomes at either -20°C or -70°C for 24 hours. After freezing, the samples were thawed to room temperature and aliquots were taken to determine a drug-to-lipid ratio for each encapsulated drug. Lipid and FUDR levels were quantified using liquid scintillation and absorbance at 370 nm was used to determine irinotecan concentration. Particle sizing of the liposomes was also determined prior to and after freezing using quasi-elastic light scattering.

Results summarized in Figure 5A show that irinotecan is effectively retained in low-cholesterol liposomes containing phosphatidylglycerol and low internal buffer osmolarity after freezing at both -20°C and -70°C for 24 hours. Further investigation into the size of these liposomes prior to and after freezing reveals that the resulting low-cholesterol liposomes comprising PG as the stabilizing lipid do not exhibit a significant change in size after freezing (Figure 5B).

Example 6

Low-cholesterol PEGylated liposomes with intraliposomal solutions of low osmolarity can be effectively frozen and thawed

The following figures demonstrate that low-cholesterol liposomes comprising PEG are resistant to aggregation upon freezing:

Liposomes consisting of various combinations of 1,2-dipalmaitoyl-sn-glycero-3-phosphocholine (DPPC), DSPC, 1,2-arachidoyl-sn-glycero-3-phosphocholine (DAPC), DSPE-PEG2000, DSPE-PEG750 and cholesterol, were prepared according to the methods of Example 1, except that lipid films were hydrated in HBS (about 320 mOsm/kg), pH 7.4 and following extrusion and size determination, the liposomes were passed through a Sephadex G50 column equilibrated in HBS, pH 7.4. The resulting liposomes were frozen in liquid nitrogen (-196°C) for 24 hours and allowed to thaw at room temperature followed by a second determination of average liposome size.

Figure 6A shows that DPPC/DSPE-PEG2000 (95:5 mole ratio) liposomes hydrated in HBS did not exhibit substantial changes in size subsequent to freezing. In contrast, DPPC/Chol (55:45 mole ratio) and DPPC/Chol/DSPE-PEG2000 (50:45:5 mole ratio) liposomes, also hydrated in HBS, exhibited substantial increases in size subsequent to freezing. Standard deviations for DPPC/DSPE-PEG2000, DPPC/Chol and DPPC/Chol/DSPE-PEG2000 liposomes prior to freezing were 27.2%, 44.8% and 19.8% respectively. After freezing, standard deviations were 24.0%, 63.6% and 64.9% for DPPC/DSPE-PEG2000, DPPC/Chol and DPPC/Chol/DSPE-PEG2000 liposomes. Chi squared values were 0.932 for DPPC/Chol liposomes and greater than 1 for DPPC/Chol/DSPE-PEG2000 liposomes subsequent to freezing.

Figure 6B shows that the size of DSPC/DSPE-PEG2000 (95:5 mole ratio) liposomes hydrated in HBS did not change subsequent to freezing whereas liposomes hydrated with HBS and consisting of DSPC/cholesterol (55:45 mole ratio) and DSPC/cholesterol/DSPE-PEG2000 (50:45:5 mole ratio) followed the same trend as in Figure 6A. The results also show that the stability of these liposomes is dramatically reduced in the absence of a stabilizing agent, such as DSPE-PEG2000. Standard deviations for DSPC/DSPE-PEG2000, DSPC/Chol and DSPC/Chol/DSPE-PEG2000 liposomes prior to freezing was 23.3%, 37.2% and 15.6% respectively. After freezing, standard deviations for DSPC/DSPE-PEG2000, DSPC/Chol and DSPC/Chol/DSPE-PEG2000 liposomes after freezing were 23.4%, 107.7% and 58.1%. For DSPC/Chol samples subsequent to freezing, chi squared values were greater than 1.

Figure 6C shows that liposomes consisting of DPPC/DSPE-PEG750 (95:5 mole ratio) and DSPC/DSPE-PEG750 (95:5 mole ratio) and hydrated in HBS also do not change in size subsequent to freezing thus demonstrating that low molecular weight hydrophilic polymers also protect against liposome aggregation due to freezing in these low-cholesterol systems. Standard deviations for DPPC/DSPE-PEG750 and DSPC/DSPE-PEG750 liposomes prior to freezing were 27.2% and 26.2% respectively. After freezing, standard deviations for DPPC/DSPE-PEG750 and DSPC/DSPE-PEG750 liposomes were 28.0% for both samples.

Figure 6D shows that liposomes consisting of DAPC/DSPE-PEG2000 (95:5 mole ratio) and hydrated in HBS also did not change in size substantially subsequent to freezing thus demonstrating that increases in acyl chain length do not affect cryostability properties. Standard deviations were 63.3 % and 50.6% for the liposomes prior to and subsequent to freezing.

Example 7

Calculating the osmolarity of an intraliposomal solution

In order to determine the osmolarity of internal liposomal solutions either prior to or after drug encapsulation, a number a techniques may be used. Preferred calculations for cholesterol-free liposomes are described below. These calculations are an extension of those previously established by Perkins et al., (Biochimica et Biophysica Acta (1988) 943(1): 103-107) for determination of the captured volume or internal volume of MLVs.

As outlined in Perkins et al., the volume of the intraliposomal solution ($V_i$) of a liposome suspension is calculated based on the partial volumes present:

$$V_T = V_0 + V_I + V_L \qquad (1)$$

Where $V_T$ is the total sample volume, $V_0$ the external aqueous volume and $V_L$ the volume occupied by the lipid. $V_L$ was calculated from the amount of lipid(s) present multiplied by its partial specific volume. $V_T$ and $V_0$ are calculated using radiolabeled water and glucose. To achieve this, lipid films are hydrated in $^3H_2O$ and after the liposomes have formed, the external aqueous volume ($V_0$) is marked by the addition of [$^{14}C$]glucose. The specific activities of each isotope in the sample are then measured and the samples centrifuged. This allows for calculation of $V_T$ and $V_0$ in the pellet after any buffer is removed. From this, $V_i$ is determined using equation 1.

Perkins et al., also developed an electron spin resonance (ESR) method as an alternative approach for calculating $V_0$. This technique uses a probe or label, such as 4-trimethylammonium TEMPO, that has minimal interaction with the liposomal membrane and thus allows for marking of the external solution exclusively. Liposome-specific probes are chosen such that they neither permeate nor bind substantially to the liposomal membrane. In order to calculate $V_0$, a known amount of label is added to the liposome sample and its concentration in the external solution is measured by using "a standard curve comparing label concentration to the amplitude of the $m_1 = +1$ resonance peak arising from the probe in buffer". By comparing the increase in label concentration measured with the concentration that would arise in the absence of the liposomes, they were able to determine the extent that the label was excluded from $V_i$ and thus derive equation 2:

$$V_0 = M/C \qquad (2)$$

Where M is the number of moles of label added and C is its measured concentration. Determination of $V_T$ by knowing the total amount of lipid(s) used to prepare the liposomes allows for $V_i$ to be calculated using equation 1. These calculations are preferable for LUVs.

Once $V_i$ has been calculated as in Perkins et al., we can use this volume to determine the osmolarity of the intraliposomal solution by measuring changes in $V_i$ due to an influx or efflux of water. To do this, we expose an aliquot of liposomes to a number of solutions with varying osmolarities and changes in the intraliposomal volume due to water movement are measured until no change occurs. At this point, the internal and external solutions are considered isotonic and thus the osmolarity of the external solution represents the osmolarity of the intraliposomal solution.

Another technique that may be used to determine the osmolarity of intraliposomal solutions includes directly measuring a large sample (> 100 μmols/mL final lipid concentration) of prepared liposomes using a freezing point osmometer (Advanced Instruments Freezing Point Osmometer Model 3D3). An aliquot of the liposomes is lysed in a low osmolarity solution, such as 1% Triton X-100 in water. The osmolarity of the solution is measured prior to and after addition of liposomes. In this way, the difference of measured osmolarities is representative of the osmolarity of the intraliposomal solution. The amount of liposomes used in the assay must be large enough (*e.g.* 100 mM lipid) to ensure that the total volume of the intraliposomal solution being measured is sufficient to generate a measurable change in the osmolarity of the external solution used to lyse the liposomes.

Example 8

The osmolarity of the hydration medium can be indicative of the osmolarity of the intraliposomal space

Alternatively, the intraliposomal osmolarity of liposomes may be determined by simply determining the osmolarity or osmolality of the solution used to hydrate lipid films during liposome preparation. This technique is preferred when the hydration solution contains components that are impermeable to the lipid bilayer and less suitable when the aqueous interior of the liposome contains salts such as NaCl and molecules such as glycerol and glucose that readily cross the liposomal membrane.

Examples of solutions that contain components that do not readily cross the liposomal membrane are given in Table I along with the measured osmolality and osmolarity values. These values were determined employing a freezing point osmometer (Advanced Instruments Freezing Point Osmometer Model 3D3) using standard solutions of NaCl.

**Table I**

| Solution | mOsm/kg or mOsm/L* |
|---|---|
| 300 mM citrate, pH 4 | 540 |
| 300 mM $MnSO_4$, 30 mM HEPES, pH 4.7 | 349 |
| 300 mM sucrose, 30 mM HEPES, pH 7.5 | 380 |
| 300 mM $MnSO_4$, pH 3.5 | 319 |
| 120 mM $(NH_4)_2SO_4$, pH 5.5 | 276 |
| 300 mM sucrose, 20 mM HEPES, 15 mM EDTA, pH 7.5 | 517 |
| 300 mM citrate, pH 7.5 adjusted with NaHCO3 | 675 |
| * Units may be interchanged between mOsm/kg or mOsm/L as aqueous solutions were employed | |

The osmolarity of various copper-containing solutions at various concentrations were measured as described above. Solutions of $CuSO_4$, Cu(II)gluconate, Cu(II)gluconate, pH 7.4 (pH adjusted with TEA), copper tartrate, pH 7.4 (pH adjusted with NaOH and HCl) were prepared at concentrations of 50, 100, 150, 200, 250 and 300 mM. Buffered Cu(II)gluconate solutions were adjusted to pH 7.4 using concentrated TEA and copper tartrate solutions were adjusted to pH 7.4 by adding NaOH until the solution was pH 12 and then adding HCl until the pH was 7.4.

Results in Figure 7 summarize the increases in measured osmolality observed with increasing concentrations of the various copper-containing solutions. The greatest increases in osmolarity with increasing concentration were observed for Cu(II)gluconate, pH 7.4 and copper tartrate, pH 7.4. Solutions of unbuffered Cu(II)gluconate and unbuffered $CuSO_4$ displayed a more modest increase in osmolarity with increasing mole concentrations of the copper salt.

**Claims**

1.   A composition comprising liposomes, wherein said liposomes comprise at least one vesicle forming lipid and at least one aggregation preventing component and contain less than 20 mol% of cholesterol,
    wherein the said liposomes contain at least one encapsulated biologically active agent; and
    wherein the intraliposomal aqueous medium has an osmolarity of 500 mOsm/kg or less.

2.   The composition of claim 1 wherein said liposomes have a transition temperature of 38°C or greater.

3.   The composition of claim 1 or 2 wherein the liposomes are large unilamellar vesicles (LUV).

EP 1 448 165 B1

4. The composition of any one of claims 1 to 3 wherein the biologically active agent comprises an antineoplastic agent.

5. The composition of any one of claims 1 to 4 wherein the intraliposomal aqueous medium has an osmolarity of 300 mOsm/kg or less.

6. The composition of any one of claims 1 to 5 wherein the vesicle forming lipid comprises a diacylphosphoglyceride wherein the acyl moieties contain at least 16 carbons.

7. The composition of any one of claims 1 to 6 wherein said intraliposomal aqueous medium comprises citrate or TEA buffer.

8. The composition of any one of claims 1 to 7 wherein the at least one aggregation preventing component includes a hydrophilic polymer-lipid conjugate.

9. The composition of any one of claims 1 to 8 wherein the vesicle forming lipids comprise distearoylphosphatidylcholine (DSPC) and wherein the aggregation preventing component comprises 10-30 mol% of a phosphatidylglycerol and the intraliposomal aqueous medium comprises 200-240 mM TEA and 100-150 mM Cu(II) gluconate.

10. The composition of any one of claims 1 to 9 wherein the biologically active agent comprises FUDR and/or CPT-11.

11. The composition of any one of claims 1 to 10 which further comprises at least one pharmaceutically acceptable excipient.

12. Use of a composition of any one of claims 1 to 11 for the preparation of a pharmaceutical composition for the treatment of cancer, cardiovascular diseases such as hypertension, cardiac arrhythmia and restenosis, bacterial, fungal or parasitic infections, inflammation or autoimmune diseases.

13. Use of a composition of any one of claims 1 to 11 for the preparation of a pharmaceutical composition as a vaccine.

14. A method of making a liposome comprising an encapsulated pH gradient loadable agent comprising the steps of:

(i) providing a liposome containing less than 20 mol% of cholesterol, said vesicle encapsulating one or more internal loading buffers having a known pH and having a concentration of less than 200 mM;
(ii) suspending said liposome in an external buffer having a pH which is different than that of the internal loading buffer whereby a pH gradient is formed across a membrane of the liposome such that the pH gradient loadable agent is neutral when present in the exterior buffer and charged when present in the internal loading buffer;
(iii) adding a pH gradient loadable agent to the mixture of (ii) and incubating the mixture for a time sufficient for uptake of the agent into the liposome.

15. A method of making a liposome comprising an encapsulated pH gradient loadable agent comprising the steps of:

(i) providing a liposome comprising:

a) from about 2 to about 30 mol% of one or more aggregation preventing agents;
b) up to about 98 mol% of one or more vesicle-forming lipids;
c) one or more internal loading buffers encapsulated within the liposome having a known pH and having a concentration of less than 200 mM;

wherein the liposome contains less than 20 mol% of cholesterol;
(ii) suspending the liposome in an external buffer having a pH which is different than that of the internal loading buffer whereby a pH gradient is formed across the membrane of the liposome such that the pH gradient loadable agent is neutral when present in the exterior buffer and charged when present in the internal loading buffer;
(iii) adding a pH gradient loadable agent to the mixture of (ii) and incubating the mixture for a time sufficient for uptake of the agent into the liposome interior.

15

**Patentansprüche**

1. Zusammensetzung, die Liposomen umfasst, wobei die Liposomen mindestens ein Vesikel-bildendes Lipid und mindestens eine Aggregations-verhindernde Komponente umfassen und weniger als 20 mol% Cholesterin enthalten, wobei die Liposomen mindestens ein eingekapseltes biologisch aktives Agens enthalten; und wobei das intraliposomale wässrige Medium eine Osmolarität von 500 mOsm/kg oder weniger hat.

2. Zusammensetzung gemäß Anspruch 1, wobei die Liposomen eine Übergangstemperatur von 38°C oder höher haben.

3. Zusammensetzung gemäß Anspruch 1 oder 2, wobei die Liposomen große unilamellare Vesikel (LUV) sind.

4. Zusammensetzung gemäß einem der Ansprüche 1 bis 3, wobei das biologisch aktive Agens ein antineoplastisches Agens umfasst.

5. Zusammensetzung gemäß einem der Ansprüche 1 bis 4, wobei das intraliposomale wässrige Medium eine Osmolarität von 300 mOsm/kg oder weniger hat.

6. Zusammensetzung gemäß einem der Ansprüche 1 bis 5, wobei das Vesikel-bildende Lipid ein Diacylphosphoglycerid umfasst, wobei die Acylreste mindestens 16 Kohlenstoffe enthalten.

7. Zusammensetzung gemäß einem der Ansprüche 1 bis 6, wobei das intraliposomale wässrige Medium Citrat- oder TEA-Puffer umfasst.

8. Zusammensetzung gemäß einem der Ansprüche 1 bis 7, wobei die mindestens eine Aggregation-verhindernde Komponente ein hydrophiles Polymer-Lipid-Konjugat einschließt.

9. Zusammensetzung gemäß einem der Ansprüche 1 bis 8, wobei die Vesikel-bildenden Lipide Distearoylphosphatidylcholin (DSPC) umfassen und wobei die Aggregation-verhindernde Komponente 10-30 mol% eines Phosphatidylglycerins umfasst und das intraliposomale wässrige Medium 200-240 mM TEA und 100-150 mM Cu(II)-gluconat umfasst.

10. Zusammensetzung gemäß einem der Ansprüche 1 bis 9, wobei das biologisch aktive Agens FUDR und/oder CPT-11 umfasst.

11. Zusammensetzung gemäß einem der Ansprüche 1 bis 10, die ferner mindestens einen pharmazeutisch verträglichen Exzipienten umfasst.

12. Verwendung einer Zusammensetzung gemäß einem der Ansprüche 1 bis 11 für die Herstellung eines Arzneimittels zur Behandlung von Krebs, kardiovaskulären Erkrankungen wie Bluthochdruck, Herzrhythmusstörung und Restenose, bakterielle, Pilz- oder parasitäre Infektionen, Entzündung oder Autoimmunerkrankungen.

13. Verwendung einer Zusammensetzung gemäß einem der Ansprüche 1 bis 11 für die Herstellung eines Arzneimittels als einen Impfstoff.

14. Verfahren zur Herstellung eines Liposoms, das ein eingekapseltes pH-Gradientenbeladbares Agens umfasst, umfassend die Schritte:

   (i) das Bereitstellen eines Liposoms, das weniger als 20 mol% Cholesterin enthält, wobei das Vesikel ein oder mehrere interne Beladungspuffer einkapselt, die einen bekannten pH-Wert und eine Konzentration von weniger als 200 mM haben;
   (ii) das Suspendieren des Liposoms in einem externen Puffer der einen pH-Wert hat, der unterschiedlich zu dem des internen Beladungspuffers ist, wobei ein pH-Gradient durch die Membran des Liposoms gebildet wird, so dass das pH-Gradienten-beladbare Agens neutral ist, wenn es in dem äußeren Puffer vorliegt, und geladen ist, wenn es in dem internen Beladungspuffer vorliegt;
   (iii) das Hinzufügen eines pH-Gradienten-beladbaren Agens zu dem Gemisch von (ii) und das Inkubieren des Gemischs für eine Zeit, die für die Aufnahme des Agens in das Liposom ausreichend ist.

**15.** Verfahren zur Herstellung eines Liposoms, das ein eingekapseltes pH-Gradientenbeladbares Agens umfasst, umfassend die Schritte:

(i) das Bereitstellen eines Liposoms, das umfasst:

(a) etwa 2 bis etwa 30 mol% von ein oder mehreren Aggregationverhindernden Agenzien;
(b) bis zu etwa 98 mol% eines oder mehrer Vesikel-bildender Lipide;
(c) ein oder mehrere interne Beladungspuffer, die in das Liposom eingekapselt sind, mit einem bekannten pH-Wert und mit einer Konzentration von weniger als 200 mM;

wobei das Liposom weniger als 20 mol% Cholesterin enthält;
(ii) das Suspendieren des Liposoms in einem externen Puffer der einen pH-Wert hat, der unterschiedlich zu dem des internen Beladungspuffers ist, wobei ein pH-Gradient durch die Membran des Liposoms gebildet wird, so dass das pH-Gradienten-beladbare Agens neutral ist, wenn es in dem äußeren Puffer vorliegt, und geladen ist, wenn es in dem internen Beladungspuffer vorliegt;
(iii) das Hinzufügen eines pH-Gradienten-beladbaren Agens zu dem Gemisch von (ii) und das Inkubieren des Gemischs für eine Zeit, die für die Aufnahme des Agens in das Liposom ausreichend ist.

**Revendications**

**1.** Composition comprenant des liposomes, dans laquelle lesdits liposomes comprennent au moins un lipide formant des vésicules et au moins un composant évitant l'agrégation et contiennent moins de 20 % en moles de cholestérol, dans laquelle lesdits liposomes contiennent au moins un agent biologiquement actif encapsulé ; et
dans laquelle le milieu aqueux intraliposomal a une osmolalité de 500 mOsm/kg
ou moins.

**2.** Composition selon la revendication 1, dans laquelle lesdits liposomes ont une température de transition de 38°C ou plus.

**3.** Composition selon la revendication 1 ou 2, dans laquelle les liposomes sont de grosses vésicules unilamellaires (LUV).

**4.** Composition selon l'une quelconque des revendications 1 à 3, dans laquelle l'agent biologiquement actif comprend un agent antinéoplasique.

**5.** Composition selon l'une quelconque des revendications 1 à 4, dans laquelle le milieu aqueux intraliposomal a une osmolalité de 300 mOsm/kg ou moins.

**6.** Composition selon l'une quelconque des revendications 1 à 5, dans laquelle le lipide formant des vésicules comprend un diacylphosphoglycéride où les parties acyles contiennent au moins 16 carbones.

**7.** Composition selon l'une quelconque des revendications 1 à 6, dans laquelle ledit milieu aqueux intraliposomal comprend un citrate ou un tampon TEA.

**8.** Composition selon l'une quelconque des revendications 1 à 7, dans laquelle le au moins un composant évitant l'agrégation comprend un conjugué polymère hydrophile - lipide.

**9.** Composition selon l'une quelconque des revendications 1 à 8, dans laquelle les lipides formant des vésicules comprennent la distéaroylphosphatidylcholine (DSPC) et dans laquelle le composant évitant l'agrégation comprend 10 à 30 % en mole d'un phosphatidylglycérol et le milieu aqueux intraliposomal comprend 200 à 240 mM de TEA et 100 à 150 mM de gluconate de Cu(II).

**10.** Composition selon l'une quelconque des revendications 1 à 9, dans laquelle l'agent biologiquement actif comprend FUDR et/ou CPT-11.

**11.** Composition selon l'une quelconque des revendications 1 à 10, qui comprend en outre au moins un excipient pharmaceutiquement acceptable.

**12.** Utilisation d'une composition selon l'une quelconque des revendications 1 à 11 pour la préparation d'une composition pharmaceutique pour le traitement du cancer, des maladies cardiovasculaires comme l'hypertension, l'arythmie cardiaque, la resténose, des infections bactériennes, fongiques ou parasitaires, de l'inflammation ou des maladies autoimmunes.

**13.** Utilisation d'une composition selon l'une quelconque des revendications 1 à 11 pour la préparation d'une composition pharmaceutique en tant que vaccin.

**14.** Procédé de fabrication d'un liposome comprenant un agent encapsulé pouvant être chargé par gradient de pH comprenant les étapes consistant à :

(i) fournir un liposome contenant moins de 20 % en mole de cholestérol, ladite vésicule encapsulant un ou plusieurs tampons de chargement internes ayant un pH connu et ayant une concentration inférieure à 200 mM ;
(ii) suspendre ledit liposome dans un tampon externe ayant un pH qui est différent de celui du tampon de chargement interne, moyennant quoi un gradient de pH est formé à travers une membrane du liposome de sorte que l'agent pouvant être chargé par gradient de pH soit neutre quand il est présent dans le tampon extérieur et chargé quand il est présent dans le tampon de chargement interne ;
(iii) ajouter un agent pouvant être chargé par gradient de pH au mélange de (ii) et incuber le mélange pendant une durée suffisante pour l'absorption de l'agent dans le liposome.

**15.** Procédé de fabrication d'un liposome comprenant un agent encapsulé pouvant être chargé par gradient de pH, comprenant les étapes consistant à :

(i) fournir un liposome comprenant :

a) d'environ 2 à environ 30 % en mole d'un ou de plusieurs agents évitant l'agrégation;
b) jusqu'à environ 98 % en mole d'un ou de plusieurs lipides formant des vésicules ;
c) un ou plusieurs tampons de chargement interne encapsulé à l'intérieur du liposome, ayant un pH connu et ayant une concentration inférieure à 200 mM ;

où le liposome contient moins de 20 % en mole de cholestérol ;
(ii) suspendre le liposome dans un tampon externe ayant un pH qui est différent de celui du tampon de chargement interne, moyennant quoi un gradient de pH est formé à travers la membrane du liposome de sorte que l'agent pouvant être chargé par gradient de pH soit neutre quand il est présent dans le tampon extérieur et chargé quand il est présent dans le tampon de chargement interne;
(iii) ajouter un agent pouvant être chargé par gradient de pH au mélange de (ii) et incuber le mélange pendant une durée suffisante pour l'absorption de l'agent à l'intérieur du liposome.

**FIGURE 1**

**FIGURE 2**

**FIGURE 3A**

**FIGURE 3B**

**FIGURE 4**

FIGURE 5A

FIGURE 5B

**FIGURE 6A**

**FIGURE 6B**

**FIGURE 6C**

**FIGURE 6D**

**FIGURE 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 60331249 B
- US 60331248 B
- US 5736155 A
- US 5077056 A
- US 5762957 A
- CA 0100655 W
- US 5013556 A
- US 5593622 A
- US 5395619 A

### Non-patent literature cited in the description

- **ALLEN et al.** *Biochim. Biophys. Acta,* 1980, 418-426
- **GABER et al.** *Pharm Res,* 1995, vol. 10, 1407-1416
- **MUI et al.** *Biophys J.,* 1993, vol. 64, 443-453
- **MUI et al.** *J. Biol. Chem.,* 1994, vol. 269, 7364-7370
- **JORGENSEN et al.** *Cell. Mol. Biol. Lett.,* 2001, vol. 6, 255-263
- **BLUME et al.** *Biochim. Biophys. Acta,* 1990, vol. 1029, 91-97
- **BLUME et al.** *Biochim. Biophys. Acta,* 1993, vol. 1146, 157-168
- **KENWORTHY et al.** *Biophys. J.,* 1995, vol. 68, 1903-1920
- **BELSITO et al.** *Biophys. J.,* 2001, vol. 93, 11-22
- **YAMAZAKI et al.** *Biophys. Chem.,* 1992, vol. 43, 29-37
- **MARUYAMA et al.** *Biochim. Biophys. Acta,* 1992, vol. 1128 (1), 44-49
- **MARUYAMA et al.** *Chem. Pharm. Bull (Tokyo,* 1991, vol. 39 (6), 1620-1622
- **BEDU-ADDO et al.** *Pharm. Res.,* 1996, vol. 13 (5), 710-717
- **PERKINS et al.** *Biochim. Biophys. Acta,* 1988, vol. 943, 103-107
- **HOPE et al.** *Biochim. Biophys. Acta,* 1984, 55-64
- **PERKINS et al.** *Biochimica et Biophysica Acta,* 1988, vol. 943 (1), 103-107